# EUROPÄISCHE PATENTSCHRIFT

(11) **EP 1 853 219 B1**
(45) Veröffentlichungstag und Bekanntmachung des Hinweises auf die Patenterteilung: **05.12.2018**
(21) Anmeldenummer: 06707200.9
(22) Anmeldetag: 23.02.2006
(51) Int. Cl.: A61K 8/31, A61Q 19/00, C25B 3/02

(54) **EMOLLIENTS UND KOSMETISCHE ZUSAMMENSETZUNGEN AUF BASIS SPEZIELLER VERZWEIGTER KOHLENWASSERSTOFFE**
EMOLLIENTS AND COSMETIC COMPOSITIONS BASED ON SPECIAL BRANCHED HYDROCARBONS
EMOLLIENTS ET COMPOSITIONS COSMETIQUES A BASE D'HYDROCARBURES RAMIFIES SPECIFIQUES

(30) Priorität: 04.03.2005 DE 102005009853; 11.03.2005 DE 102005011691
(43) Veröffentlichungstag der Anmeldung: 14.11.2007
(73) Patentinhaber: Cognis IP Management GmbH, 40589 Düsseldorf (DE)
(72) Erfinder: DIERKER, Markus, 40597 Düsseldorf (DE)
(74) Vertreter: BASF IP Association
(86) Internationale Anmeldenummer: PCT/EP2006/001641
(87) Internationale Veröffentlichungsnummer: WO 2006/094642

(56) Entgegenhaltungen:
- EP-A- 1 512 392
- US-A- 2 680 713
- US-A- 2 760 926
- US-A- 4 853 217
- US-A- 5 627 056
- PATENT ABSTRACTS OF JAPAN Bd. 010, Nr. 345 (C-386), 20. November 1986 (1986-11-20) & JP 61 147890 A (OKAMURA SEIYU KK), 5. Juli 1986 (1986-07-05)

## Beschreibung

### Gebiet der Erfindung

Die Erfindung betrifft kosmetische Zusammensetzungen mit Ölkörpern auf Kohlenwasserstoffbasis, die mittels Kolbe-Synthese erhalten werden sowie die Verwendung dieser Kohlenwasserstoffe.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet.

Der Einsatz von Kohlenwasserstoffen in kosmetischen Zusammensetzungen ist seit langem bekannt. So werden beispielsweise Mineralöle und Paraffinöle als inerte Ölkörper eingesetzt. Diese haben den Nachteil, dass sie aus sensorischer Sicht ein "schweres" Hautgefühl hinterlassen und schlecht spreiten. Bekannt ist auch der Einsatz von Produktmischungen verschiedenster Kohlenwasserstoffe mit verbesserter Spreitfähigkeit, wie sie beispielsweise gemäß der in DE 103 17 781 oder DE 103 24 508 beschriebenen Verfahren erhältlich sind. Derartige Gemische sind nur schwer charakterisierbar und enthalten eine Vielzahl verschiedener Einzelkomponenten, deren einzelner Beitrag zur Spreitfähigkeit nur schwer ermittelbar ist.

Aufgabe der vorliegenden Erfindung war es daher, alternative hochspreitende, volatile Kohlenwasserstoffe für den Einsatz in der Kosmetik zur Verfügung zu stellen, die sich einfach und in hoher Ausbeute herstellen lassen. Ein weiterer Teilaspekt war es, Kohlenwasserstoffe zur Verfügung zu stellen, die sich als Silikonersatz eignen, also das sensorische Profil von flüchtigen Silikonen aufweisen.

Überraschenderweise wurde gefunden, dass Kohlenwasserstoffe, die durch Kolbe-Elektrolyse aus Fettsäuren hergestellt wurden, sich als volatile, hochspreitende Emollients für den Einsatz in der Kosmetik eignen und spezifisch in hoher Ausbeute erhalten werden. Die Kolbe-Elektrolyse selbst ist eine seit langem bekannte Methode zur Herstellung von Kohlenwasserstoffen (H. Kolbe, Liebigs Ann. Chem. 1849, 69, 257 - 294) und als solches Stand der Technik. Die damit gezielt herstellbarem Kohlenwasserstoffe, die symmetrisch aufgebaut sind, wurden bisher allerdings nicht für den Einsatz in der Kosmetik beschrieben.

### Beschreibung der Erfindung

Gegenstand der Erfindung sind daher kosmetische Zusammensetzungen gemäß Anspruch 1. Erfindungsgemäß muß der Kohlenwasserstoff eine Kettenlänge von wenigstens 10 Kohlenstoffatomen haben, um für kosmetische Applikationen geeignet zu sein. Im Sinne der Erfindung kann die kosmetische oder pharmazeutische Zusammensetzung sowohl ein einzigen Kohlenwasserstoff enthalten, der durch Kolbe-Elektrolyse erhalten wurde, als auch ein Produkt-Gemisch. Was im Einzelfall vorteilhaft ist, hängt vom jeweiligen Applikationszweck ab.

Der Vorteil der Kolbe-Elektrolyse ist, dass es nicht zur Isomerisierung der Radikale kommt. Die MetallKationen wandern unter dem Einfluss des Gleichstroms zur Kathode, die negativ geladenen Carboxylate zur Anode, an der durch anodische Oxidation eine Decarboxylierung eintritt. Die entstehenden AlkylRadikale dimerisieren anschließend. Die durch Dimersierung erhaltenen Produkte sind symmetrisch und haben Strukturen, in denen sich das Strukturmerkmal der Fettsäure exakt abbildet, d.h. erhalten bleibt. Die durch Kolbe-Elektrolyse erhaltenen Emollients weisen somit ein Substitutionsmuster auf, das durch andere Herstellmöglichkeiten (z.B. Oligomerisierung) nicht erhältlich ist. Das unterscheidet die durch Kolbesynthese erhaltenen Alkane von bereits bekannten verzweigten Alkan-Emollients. So führt beispielsweise die Kolbe-Elektroylse von 2-Ethylhexan spezifisch zum symmetrisch aufgebauten Diethyldecan (J.E. Barry, M. Finkelstein, E.A. Mayeda, S.D. Ross, J. Am. Chem. Soc. 1976, 98, 8098-8101).

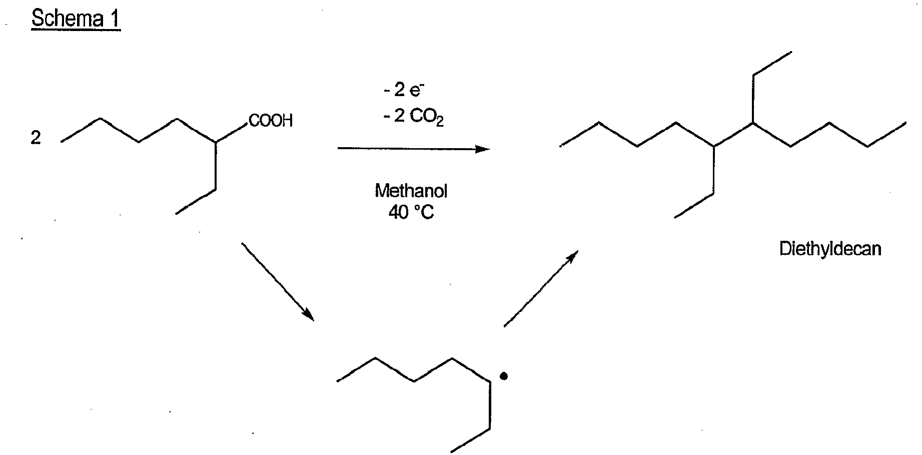

Je nachdem, ob man als Ausgangsstoff eine einzige Fettsäure oder ein Fettsäuregemisch einsetzt, läßt sich die Anzahl der zu erwartenden Produkte genau steuern. Bei einer Elektrolyse von Carbonsäure-Gemischen erhält man alle möglichen Produkte symmetrischer und unsymmetrischer Radikal-Kombinationen. Anders als bei Kohlenwasserstoff-Gemischen, die durch Kondensationsreaktionen und Polymerisierungen erhalten werden, ist die Zusammensetzung des Produktgemisches bei der Kolbe-Elektrolyse leichter steuerbar, genau definiert und weit weniger komplex. Damit lassen sich auch gezielt Verzweigungsstellen in die Kohlenwasserstoffe einbauen und Kohlenwasserstoffgemische mit optimierten Spreitkaskaden entwickeln, deren Einsatz in der Kosmetik zu sensorischen Vorteilen führt. Erfindungsgemäß kann beispielsweise ein Gemisch aus Pivalinsäure und Isostearinsäure im Molverhältnis von 1:1 der Kolbe-Elektrolyse unterworfen werden und das Reaktionsprodukt in kosmetische Formulierungen eingearbeitet werden.

Erfindungsgemäß besonders bevorzugt sind Zusammensetzungen, die dadurch gekennzeichnet sind, dass der durch Kolbe-Elektrolyse hergestellte Kohlenwasserstoff höchstens 22 Kohlenstoffatome aufweist. Kohlenwasserstoffe mit höchstens 22 Kohlenstoffatomen zeichnen sich durch eine bessere Flüchtigkeit und sensorische Vorteile in den Endformulierungen aus und eignen sich besonders gut als Silikonersatz.

Die erfindungsgemäßen Zusammensetzungen enthalten die durch Kolbe-Elektrolyse hergestellten Kohlenwasserstoffe vorzugsweise in einer Menge von 0,1 - 50 Gew.-% bezogen auf die Gesamtzusammensetzung.

Gegenstand der Erfindung ist auch die Verwendung gemäß Anspruch 3 als hochspreitende Ölkörper oder als Silikonersatz in kosmetischen Zusammensetzungen.

Ein weiterer Gegenstand der Anmeldung ist ein Verfahren zur Herstellung von kosmetischen und/oder pharmazeutischen Zusammensetzungen, gemäß Anspruch 4 wobei man einen Kohlenwasserstoff mit wenigstens 10 Kohlenstoffatomen, als Ölphase, die gegebenenfalls weitere öllösliche Komponenten enthält, zusammen mit einer Wasserphase, die gegebenenfalls weitere wasserlösliche Komponenten enthält, und gegebenenfalls weiteren üblichen Hilfs- und Zusatzstoffen zu einer Emulsion verarbeitet. Vorzugsweise wird die Kolbe-Elektroylse mit unverdünnten Fettsäuren oder mit Fettsäuren in einem Lösungsmittel durchführt, wobei 1 - 25 Mol-% der Fettsäuren mit einer Base neutralisiert werden und die Elektrolyse bei Stromdichten von 100 -1000 mA/cm² und einer Temperatur von 0 - 70°C durchgeführt wird.

### Kolbe-Elektrolyse (allgemeine Vorschrift)

Die Fettsäuren werden rein oder in einem Lösungsmittel (vorzugsweise Methanol; besonders bevorzugt Methanol mit Wasser (1-5 Gew.-%, bevorzugt 2,5 - 3 Gew.-%) eingesetzt. Ein Teil der Säure (1-25 Mol-%, vorzugsweise 5-15 Mol-%) wird mit Base neutralisiert (bevorzugte Base ist NatriumMethanolat). Die Elektrolyse wird an Platin-, Platin/Niob-, Graphit- oder Glaskohlenstoff- (glassy carbon)-Elektroden durchgeführt. Die Stromdichte beträgt 100-1000 mA/cm² (vorzugsweise 150-700 mA/cm², besonders bevorzugt 200-600 mA/cm²). Die Elektrolyse wird bei 0-70°C (bevorzugt 35-50°C) durchgeführt.

Da die resultierenden Kohlenwasserstoffe nicht mit der polaren Reaktionslösung (Carbonsäure in Methanol) mischbar sind, scheiden sich die Produkte in einer zweiten Phase ab. Diese Phase kann abgetrennt werden und enthält den gewünschten Kohlenwasserstoff (Emollient) in hoher Reinheit.

Die durch Kolbe-Elektrolyse hergestellten Kohlenwasserstoffe weisen ein besseres Spreitvermögen auf als vergleichbare Kohlenwasserstoffe (vgl. Graphik 1). Hierzu wurde ein Tropfen von 25 µl bei 24°C und 40 % Luftfeuchtigkeit auf ein Filterpapier getropft. Die selbsttätige Ausbreitung des Tropfens wird gegen die Zeit in einem Diagramm aufgetragen. Je schneller sich der Tropfen ausbreitet, umso besser ist die Spreitung.

### Kosmetische/pharmazeutische Zubereitungen

Die durch Kolbe-Elektrolyse hergestellten Kohlenwasserstoffe erlauben die Herstellung stabiler kosmetischer und pharmazeutischer Emulsionen, ggf. mit Spreitkaskaden, und eignen sich auch als Silikonersatz in kosmetischen und pharmazeutischen Zusammensetzungen.

Die erfindungsgemäßen Zusammensetzungen können Formulierungen zur Körperpflege sein, z. B. eine Körpermilch, Cremes, Lotionen, sprühbare Emulsionen, Produkte zur Eliminierung des Körpergeruchs etc. Die Kohlenwasserstoffe lassen sich auch in tensidhaltigen Formulierungen wie z. B. Schaum- und Duschbädern, Haarshampoos und Pflegespülungen einsetzen. Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antichuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

### Tenside

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 1 bis 30, vorzugsweise 5 bis 25 und insbesondere 10 bis 20 Gew.-%.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel, wie Cremes, Lotionen und Milchen, enthalten üblicherweise eine Reihe weiterer Ölkörper und Emollients, die dazu beitragen, die sensorischen Eigenschaften weiter zu optimieren. Die Ölkörper sind üblicherweise in einer Gesamtmenge von 1 - 50 Gew.-%, vorzugsweise 5 - 25 Gew.-% und insbesondere 5 - 15 Gew.-% enthalten. Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C_{3B}-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dica-prylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen. Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Perlglanzwachse**, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Beispiele

### Beispiel 1:

Die Elektrolyse wurde in einer ungeteilten 200 ml Becherglaszelle durchgeführt. Zwei Platinblech Elektroden mit einer Fläche von je 1 cm² wurden so positioniert, dass der Abstand zwischen den Elektroden 1 - 3 mm betrug. Die Elektroden wurden an ein Netzteil (3A/30V) angeschlossen. In die Zelle wurden 40 g (0,28mol) Ethylhexansäure, 2,5 g Natriummethanolat (0, 014 mol, 30 %-ig in Methanol) und 4,2 g Wasser gegeben und mit Methanol auf 150 ml aufgefüllt. Unter Rühren wurde die Lösung bei einem konstanten Strom von 0,5 A elektrolysiert, die dafür nötige Spannung betrug 26 V. Die Zelle wurde mit Eis/Wasser gekühlt, so dass sich eine Temperatur von 40°C einstellte. Nach 15 h wurde die Elektrolyse abgebrochen. Das farblose Produkt schied sich als zweite Phase ab. Die Ausbeute beträgt 16g. Die gaschromatographische Untersuchung des Produktes ergab einen Gehalt von > 95% Diethyldecan (siehe Schema).

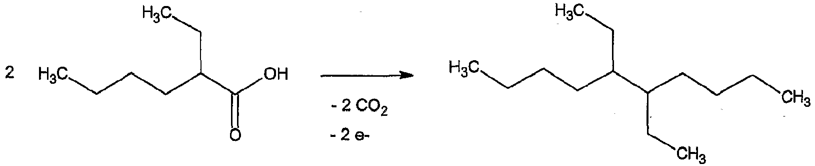

### Beispiel 2:

Die Elektrolyse wurde in einer ungeteilten 200 ml Becherglaszelle durchgeführt. Zwei Platinblech Elektroden mit einer Fläche von je 1 cm² wurden so positioniert, dass der Abstand zwischen den Elektroden 1 - 3 mm betrug. Die Elektroden wurden an ein Netzteil (max. 3A und 30 V) angeschlossen. In die Zelle wurden 40g (0,28mol) Cekanoic C8 (Fa. Exxon), 2,5 g Natriummethanolat (0, 014 mol, 30.%-ig in Methanol) und 4,2 g Wasser gegeben und mit Methanol auf 150 ml aufgefüllt. Unter Rühren wurde die Lösung bei einem konstanten Strom von 0,5 A elektrolysiert, die dafür nötige Spannung betrug 26 V. Die Zelle wird mit Eis/Wasser gekühlt, so dass sich eine Temperatur von 40°C einstellte. Nach 15 h wurde die Elektrolyse abgebrochen. Das farblose Produkt schied sich als zweite Phase ab. Die gaschromatographische Untersuchung des Produktes ergab einen Gehalt von > 95% verzweigtem C14-Kohlenwasserstoff aus mehreren Isomeren mit Methylverzweigungen. Diese Methylverzweigungen haben das gleiche Verteilungsmuster wie im Ausgangsstoff Cekanoic C8. Die Ausbeute beträgt 17,0 g.

### Beispiel 3: (illustrativ)

Die Elektrolyse wurde in einer ungeteilten 200 ml Becherglaszelle durchgeführt. Zwei PlatinblechElektroden mit einer Fläche von je 1 cm² wurden so positioniert, dass der Abstand zwischen den Elektroden 1 - 3 mm betrug. Die Elektroden wurden an ein Netzteil (max. 3A und 30 V) angeschlossen. In die Zelle wurden 23 g (0,16mol) Ethylhexansäure, 18,5 g (0,16 mol) Hexansäure, 2,9 g Natriummethanolat (0,016 mol, 30%-ig in Methanol) und 4,2 g Wasser gegeben und mit Methanol auf 150 ml aufgefüllt. Unter Rühren wurde die Lösung bei einem konstanten Strom von 0,4 A elektrolysiert, die dafür nötige Spannung betrug 26 V. Die Zelle wurde mit Eis/Wasser gekühlt, so dass sich eine Temperatur von 40°C einstellte. Nach 15 h wurde die Elektrolyse abgebrochen. Das farblose Produkt schied sich als zweite Phase ab. Die Ausbeute beträgt 18,5 g. Die gaschromatographische Untersuchung des Produktes ergab ein Gemisch aus drei verschiedenen Kohlenwasserstoffen mit einem Gesamtgehalt von > 95%. Die drei Kohlenwasserstoffe sind Dekan, Ethyldecan und Diethyldecan. (siehe Schema)

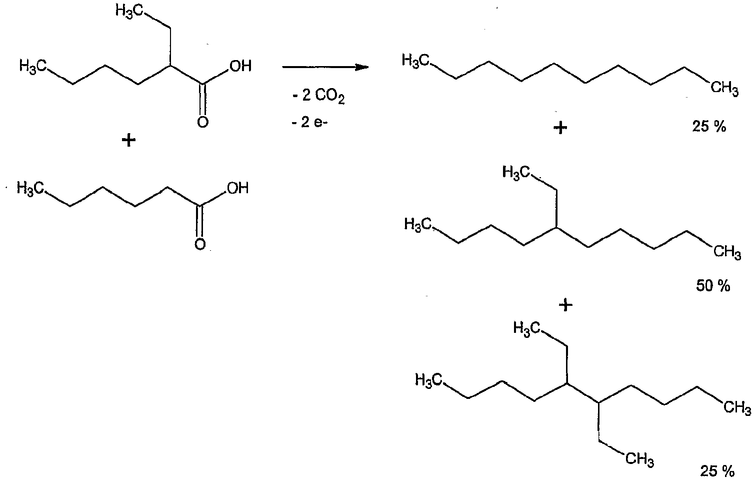

Die in den Beispielen genannten Ausbeuten sind nicht optimiert und lassen sich durch routinemäßige Optimierung des Fachmanns noch verbessern.

### Kosmetische Zusammensetzungen

Alle Mengenangaben in Gew.-% bezogen auf das Gewicht der kosmetischen Zubereitung

| **Handelsname** | **INCI Bezeichnung** | **Gew.-%** |
|---|---|---|
| | | |
| Emulgade PL68/50 | Cetearyl Glucoside (and) Cetearyl Alcohol | 5,00 |
| Amphisol K | Potassium Cetyl Phosphate | 0,50 |
| Cutina GMS-V | Glyceryl Stearate | 1,00 |
| Diethyldecan | - | 6,00 |
| Myritol 318 | Caprylic/Capric Triglyceride | 5,00 |
| Novata AB | Cocoglycerides | 1,00 |
| Wacker Siliconöl AK350 | Dimethicone | 0,30 |
| Carbopol 980 | Carbomer | 0,30 |
| Glycerin 99%ig | Glycerine | 5,00 |
| KOH (20%ig) | | 0,60 |
| Formalinlsg 37,5%ig | | 0,15 |
| H₂O dest. | | ad 100 |
| | | |
| Viskosität [mPas] *) | | 12 5000 |

| **Handelsname** | **INCI Bezeichnung** | **GEW.-%** |
|---|---|---|
| | | |
| Cutina GMS-SE | Glyceryl Stearate | 5,50 |
| Eumulgin B1 | Ceteareth-12 | 0,20 |
| Cutina FS45 | Stearic Acid (and) Palmitic Acid | 2,00 |
| Lanette 16 | Cetyl alcohol | 1,50 |
| Fitoderm | Squalane | 6,00 |
| Diethyldecan | - | 5,00 |
| Cegesoft SH | Shorea Stenoptera | 5,00 |
| Cegesoft PFO | Passiflora Incarnata | 1,00 |
| Cetiol MM | Myristyl Myristate | 1,00 |
| Glycerin 99%ig | Glcerine | 5,00 |
| Formalinlsg 37,5%ig | | 0,15 |
| H₂O dest. | | 93,85 |
| | | |
| Viskosität [mPas] *) | | 18 7500 |

| | | |
|---|---|---|
| *) Bestimmt mit TE-Spindel mit 4 Upm + Helipath bei 23 °C | | |

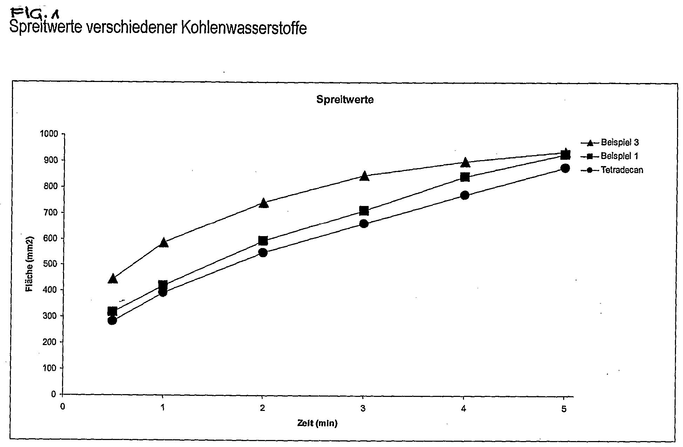

## Patentansprüche

1. Kosmetische und/oder pharmazeutische Zusammensetzung enthaltend wenigstens einen Kohlenwasserstoff mit wenigstens 10 Kohlenstoffatomen, der durch Kolbe-Elektrolyse einer verzweigten Fettsäure erhalten wird, wobei als Fettsäure Ethylhexansäure, Isononansäure, Isodecansäure, Isostearinsäure, Isooctansäure, Neodecansäure oder eine beliebige Mischung dieser Fettsäuren eingesetzt wird, oder eine Mischung der vorgenannten Fettsäure(n) mit Pivalinsäure oder Cyclohexancarbonsäure.

2. Zusammensetzung gemäß Anspruch 1, **dadurch gekennzeichnet, dass** der Kohlenwasserstoff in einer Menge von 0,1 - 50 Gew.-% bezogen auf die Gesamtzusammensetzungen enthalten ist.

3. Verwendung von Kohlenwasserstoffen mit wenigstens 10 Kohlenstoffatomen, die durch Kolbe-Elektrolyse von einer verzweigten Fettsäure erhalten werden, wobei als Fettsäure Ethylhexansäure, Isononansäure, Isodecansäure, Isostearinsäure, Isooctansäure, Neodecansäure oder eine beliebige Mischung dieser Fettsäuren eingesetzt wird oder eine Mischung der vorgenannten Fettsäure(n) mit Pivalinsäure oder Cyclohexancarbonsäure,.als hochspreitende Ölkörper oder als Silikonersatz in kosmetischen und/oder pharmazeutischen Zusammensetzungen.

4. Verfahren zur Herstellung von kosmetischen und/oder pharmazeutischen Zusammensetzungen, wobei man einen Kohlenwasserstoff mit wenigstens 10 Kohlenstoffatomen, der durch Kolbe-Elektrolyse einer verzweigten Fettsäure erhalten wird, wobei als Fettsäure Ethylhexansäure, Isononansäure, Isodecansäure, Isostearinsäure, Isooctansäure, Neodecansäure oder eine beliebige Mischung dieser Fettsäuren eingesetzt wird, oder eine Mischung der vorgenannten Fettsäure(n) mit Pivalinsäure oder Cyclohexancarbonsäure, als Ölphase, die gegebenenfalls weitere öllösliche Komponenten enthält, zusammen mit einer Wasserphase, die gegebenenfalls weitere wasserlösliche Komponenten enthält, und gegebenenfalls weiteren üblichen Hilfs- und Zusatzstoffen zu einer Emulsion verarbeitet.

5. Verfahren gemäß Anspruch 4, **dadurch gekennzeichnet, dass** man die Kolbe-Elektrolyse mit unverdünnten Fettsäuren oder mit Fettsäuren in einem Lösungsmittel durchführt, wobei 1 - 25 Mol-% der Fettsäuren mit einer Base neutralisiert werden und die Elektrolyse bei Stromdichten von 100 -1000 mA/cm² und einer Temperatur von 0 - 70° C durchgeführt wird.

## Claims

1. A cosmetic and/or pharmaceutical composition comprising at least one hydrocarbon having at least 10 carbon atoms, which is obtained by Kolbe electrolysis of a branched fatty acid, wherein the fatty acid used is ethylhexanoic acid, isononanoic acid, isodecanoic acid, isostearic acid, isooctanoic acid, neodecanoic acid or any desired mixture of these fatty acids, or a mixture of the abovementioned fatty acid(s) with pivalic acid or cyclohexanecarboxylic acid.

2. The composition according to claim 1, wherein the hydrocarbon is present in an amount of 0.1-50 wt% based on the total composition.

3. The use of hydrocarbons having at least 10 carbon atoms, which are obtained by Kolbe electrolysis of a branched fatty acid, wherein the fatty acid used is ethylhexanoic acid, isononanoic acid, isodecanoic acid, isostearic acid, isooctanoic acid, neodecanoic acid or any desired mixture of these fatty acids, or a mixture of the abovementioned fatty acid(s) with pivalic acid or cyclohexanecarboxylic acid, as high-spreading oil bodies or as silicone replacement in cosmetic and/or pharmaceutical compositions.

4. A method for preparing cosmetic and/or pharmaceutical compositions, wherein a hydrocarbon having at least 10 carbon atoms, which is obtained by Kolbe electrolysis of a branched fatty acid, wherein the fatty acid used is ethylhexanoic acid, isononanoic acid, isodecanoic acid, isostearic acid, isooctanoic acid, neodecanoic acid or any desired mixture of these fatty acids, or a mixture of the abovementioned fatty acid(s) with pivalic acid or cyclohexanecarboxylic acid, as oil phase, which optionally comprises further oil-soluble components, together with an aqueous phase, which optionally comprises further water-soluble components, and optionally further customary aids and auxiliaries, are processed to give an emulsion.

5. The method according to claim 4, wherein the Kolbe electrolysis is carried out with undiluted fatty acids or with fatty acids in a solvent, wherein 1-25 mol% of the fatty acids are neutralized with a base and the electrolysis is carried out at current densities of 100-1000 mA/cm² and a temperature of 0-70°C.

## Revendications

1. Composition cosmétique et/ou pharmaceutique, contenant au moins un hydrocarbure contenant au moins 10 atomes de carbone, qui est obtenu par électrolyse de Kolbe d'un acide gras ramifié ; l'acide éthylhexanoïque, l'acide isononanoïque, l'acide isodécanoïque, l'acide isostéarique, l'acide isooctanoïque, l'acide néodécanoïque ou un mélange quelconque de ces acides étant utilisé en tant qu'acide gras, ou un mélange du ou des acides gras susmentionnés avec de l'acide pivalique ou de l'acide cyclohexanecarboxylique.

2. Composition selon la revendication 1, **caractérisée en ce que** l'hydrocarbure est contenu en une quantité de 0,1 à 50 % en poids, par rapport aux compositions totales.

3. Utilisation d'hydrocarbures contenant au moins 10 atomes de carbone, qui sont obtenus par électrolyse de Kolbe d'un acide gras ramifié ; l'acide éthylhexanoïque, l'acide isononanoïque, l'acide isodécanoïque, l'acide isostéarique, l'acide isooctanoïque, l'acide néodécanoïque ou un mélange quelconque de ces acides étant utilisé en tant qu'acide gras, ou un mélange du ou des acides gras susmentionnés avec de l'acide pivalique ou de l'acide cyclohexanecarboxylique ; en tant que corps huileux s'étalant facilement ou en tant que substitut de silicone dans des compositions cosmétiques et/ou pharmaceutiques.

4. Procédé de fabrication de compositions cosmétiques et/ou pharmaceutiques, selon lequel un hydrocarbure contenant au moins 10 atomes de carbone, qui est obtenu par électrolyse de Kolbe d'un acide gras ramifié ; l'acide éthylhexanoïque, l'acide isononanoïque, l'acide isodécanoïque, l'acide isostéarique, l'acide isooctanoïque, l'acide néodécanoïque ou un mélange quelconque de ces acides étant utilisé en tant qu'acide gras, ou un mélange du ou des acides gras susmentionnés avec de l'acide pivalique ou de l'acide cyclohexanecarboxylique, en tant que phase huileuse, qui contient éventuellement d'autres composants solubles dans les huiles, est transformé en une émulsion conjointement avec une phase aqueuse, qui contient éventuellement d'autres composants solubles dans l'eau, et éventuellement d'autres adjuvants et additifs usuels.

5. Procédé selon la revendication 4, **caractérisé en ce que** l'électrolyse de Kolbe est réalisée avec des acides gras non dilués ou avec des acides gras dans un solvant, 1 à 25 % en moles des acides gras étant neutralisés avec une base, et l'électrolyse étant réalisée à des densités de courant de 100 à 1 000 mA/cm² et à une température de 0 à 70 °C.
